# EUROPEAN PATENT APPLICATION

(11) **EP 3 974 012 A1**
(43) Date of publication of application: **30.03.2022**
(21) Application number: 20198844.1
(22) Date of filing: 29.09.2020
(51) Int. Cl.: A61M 5/315, G16H 20/17, G16H 50/30, G16H 50/70

(54) **PRIMING EVENT IDENTIFICATION IN DELIVERY DEVICES**

(71) Applicant: Ypsomed AG, 3401 Burgdorf (CH)
(72) Inventor: Schüpbach, Simon, 3400 Burgdorf (CH); Baumgartner, Adrian, 2562, Port (CH)
(74) Representative: Meier Obertüfer, Jürg

(57) **Abstract**

The invention is concerned with a method of monitoring a use cycle or delivery process including at least a first and a second dispense event performed by means of a variable-dose delivery device. The method comprises, by an electronic unit associated with the delivery device, determining, during the use cycle, first and second event details of the first and second dispense event, respectively. The dispense event details include at least one of a start, end, or other time of the dispense event, a dispense dose dispensed during the dispense event, and a device orientation of the device during the dispense event. The method comprises determining, at the end of the use cycle, cycle properties including at least one of a number of dispense events in the use-cycle and a holding time status indicative of a regular holding time having been observed at the end of the use cycle. The method comprises evaluating, after the use cycle, an injection criteria to derive an injection, or non-priming, score of the second event, indicative of a probability that the second dispense dose has been injected into the target tissue and was not a priming or air shot. The injection criteria either is a multi-event-criteria based on an event detail of the first event, or a cycle criteria based on a cycle property of the use cycle. The method comprises determining and forwarding a cycle dispense dose based on the injection score, in particular a cycle injection dose disregarding any event dispense dose of a dispense event with a low or insufficient injection score.

## Description

### FIELD OF THE INVENTION

The present invention relates to injection devices or medicament delivery devices for injecting, delivering, administering, infusing or dispensing substances and/or liquids such as insulin or hormone preparations. It departs from an electronic unit incorporated in, or attachable to, an injection device, and comprising a sensor for monitoring an injection process executed by means of the injection device.

### BACKGROUND OF THE INVENTION

A variety of diseases exist that require regular treatment by parenteral subcutaneous or intra-muscular administration of a drug or medicament, and a number of drug delivery devices have been developed to support a patient in accurately and controllably delivering an amount of drug in a self-administration process. Delivery devices include injection devices that are removed from the injection site after each drug delivery event or medication process, as well as infusion devices with a cannula or needle that remains in the skin of the patient for a prolonged period of time. Disposable delivery devices are adapted to deliver a drug from a container such as a pre-filled syringe that is not intended to be replaced or refilled by the patient. Reusable, semi-reusable, or hybrid delivery devices have a container that may be replaced by the patient, or a cartridge that may be refilled, while some components of the device may be reused with the replaced or refilled drug container.

By way of example, diabetes may be treated by self-administration of insulin with the help of multi-variable-dose insulin injection pens. An injection pen device generally has an elongate device body defining a longitudinal main device axis. The term "distal end" refers to the end of the injection device where an injection needle is located, the term "proximal end" designates the opposite end thereof. An automatic injection device has a motor or a drive spring for biasing a piston rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The insulin dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. In order to monitor the injection of insulin, for instance to prevent false handling of the insulin pen or to keep track of the doses already applied, it is desirable to measure and process information related to a use of the injection device, such as information on the injected insulin type, dose, and circumstances of an injection process.

Drug delivery device based therapies generally benefit from an electronic unit or control unit embedded or integrated in the delivery device, or being part of an auxiliary or supplemental electronic module or add-on device detachably attached to the delivery device. The electronic unit monitors a drug delivery process, in order to proactively prevent false handling of the device and/or to keep track of the doses already applied, and generates data related to an instantaneous condition and/or use of the delivery device. Suitable sensors of the electronic unit readily detect a status or signal from any kind of indicating component of the delivery device, including user interface elements and actuators. A wireless communication unit of the electronic unit is provided to wirelessly communicate, specifically upload, drug delivery information to a nearby mobile device or dedicated medical gateway. The drug delivery information includes at least a time stamp and the expelled dose, indicative of a time of a medication event and of a quantity of delivered medicament. The drug delivery information may be transmitted instantaneously, or stored in a memory unit connected to the processing unit, for later upload or batch transfer.

WO 2019224626 A1 discloses an auxiliary electronic module for a variable-dose injection device generating a number of click sounds proportionate to a dose of medicament. The electronic module comprises sensor elements including one or more of an acceleration sensor, a gyroscope sensor, a piezoelectric contact microphone sensor, and a force or pressure transducer, for sensing tactile and/or acoustic vibrations that are generated in the injection device in the form of individual clicks for each dose unit during dialing and/or dispensing of a dose. From a signal of the sensor elements recorded as a device feedback sample signal or suspected dose-unit click sound, multiple features or characteristics are derived, such as a sum of correlation values of a test signal with single-axis gyroscope signals, or a sum of absolute derivatives of single-axis gyroscope signals. These features are evaluated in a scatter plot of a multi-dimensional feature space or otherwise classified by a classifier to assign the device feedback sample signal to a device feedback class such as a Dial-Up Click, a Dial-Down Click, an Injection Click, or a Non-Click feedback. Various types of classifiers exist and may be employed, such as a Support Vector Machine (SVM) with a linear kernel, a Feed Forward Artificial Neural Network (ANN), or a Gradient Boost Decision Tree Neural Network.

The aforementioned as well as other prior art approaches that focus on a detection of a set dose or of a dispensed dose in variable-dose injection devices by way of identifying and counting individual dosage units also require to identify priming or air shot events. A dispensed dose is to be identified as a priming dose in order to avoid reporting the priming dose as an injected dose, and to ensure that only the amounts of medication actually received by the patient are logged.

EP 2401006 A1 discloses a drug delivery pen with an inertial sensor and a microprocessor configured to determine from output signals of the inertial sensor whether the pen housing including the cartridge is oriented topmost and generally vertically with respect to the ground in a priming position. This teaching is based on a widespread user instruction paradigm promoting to orient the drug delivery pen vertically and to dispense two or three units in a priming operation. However, in a not quite uncommon act of non-conformant user handling, a user may prime with the delivery pen orientation differing to some extent from vertical, or the user may inject the liquid into his body with the delivery pen oriented approximately vertically. For both these cases, the distinction between priming and injecting based on the verticality of the pen alone is not successful.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

### SUMMARY OF THE INVENTION

It is an objective of the invention to monitor an injection process or medication event executed by means of a variable dose injection device, and specifically to accurately determine the size of a dose received by the patient in a single use cycle or delivery cycle. These objectives are achieved by a method of monitoring a use cycle of a variable-dose delivery device and by a delivery system including an electronic unit associated with a variable-dose delivery device according to the independent claims. Preferred embodiments are evident from the dependent patent claims.

According to the invention, a method of monitoring a use cycle or delivery process including at least a first and a second dispense event performed by means of a variable-dose delivery device comprises, by an electronic unit associated with the delivery device, determining, during the use cycle, first and second event details of the first and second dispense event, respectively. The dispense event details include at least one of a start, end, or other time of the dispense event, a dispense dose dispensed during the dispense event, and a device orientation of the device during the dispense event. The method comprises determining, at the end of the use cycle, cycle properties including at least one of a number of dispense events in the use-cycle and a holding time status indicative of a regular holding time having been observed at the end of the use cycle. The method comprises evaluating, after the use cycle, an injection criteria to derive an injection, or non-priming, score of the second event, indicative of a probability that the second dispense dose has been injected into the target tissue and was not a priming or air shot. The injection criteria either is a multi-event-criteria based on, in excess of the second event details, an event detail of the first event, or a cycle criteria based on a cycle property of the use cycle. The method comprises determining and forwarding a cycle dispense dose based on the injection score, in particular a cycle injection dose disregarding any event dispense dose of a dispense event with a low or insufficient injection score. The proposed injection criteria take into account sensor output produced by a sensor of the electronic unit beyond a mere device orientation at a time of the dispense event. Reverting to an accumulated probability reflecting a plurality of available criteria or conditions increases chances of distinguishing correctly between injection events and priming events, in particular in cycle sequences including more than two dispense events.

The use cycle or delivery process is monitored by means of an electronic unit associated with the delivery device. The electronic unit is part of an electronic variable-dose delivery device or of an electronic module or auxiliary device for removable attachment to a mechanical variable-dose delivery device. The variable-dose delivery device includes a reservoir or liquid drug container that may be replaced or refilled by the patient. The electronic unit comprises a device cap detector, a device movement sensor, and/or a touch sensor and is adapted and configured to identify a use cycle as being delimited by a device cap being removed from and re-attached to the delivery device, by the delivery device being seized and put down by the user, and/or by prolonged periods of device inactivity. The electronic unit is further configured to identify dispense or expel events within the use cycle, wherein every dispense event includes an initial dose dialing activity, and wherein successive dispense events are separated by a non-dispense interval of a duration considerably exceeding a regular intra-event delay between two successive dose-unit dispense clicks, and/or separated by considerable movement of the delivery device. The electronic unit is adapted and configured to determine dispense data including the amount or value of a dispense dose that is dispensed during a dispense event, and to determine the device orientation assumed by the delivery device during the dispense event. The determined dispense data are recorded along with a dispense time consistently indicating a time, such as the beginning or end, of the dispense event.

The device orientation of the delivery device is inferred from sensor data of an accelerometer and/or a gyroscope of the electronic unit, as provided during the dispense event. The sensor data is suitably averaged or filtered, and device orientations of distinct dispense events are evaluated or checked for approximate or substantial identity only. By way of example, the device orientation may be limited to a coarse identification of an upright orientation if the delivery device remains constrained in a priming cone around the axis of gravity. The device orientation qualifies as upright if it deviates, on average and, to filter accidental device orientation excursions, during a minimal event time of 0.5 seconds, by less than 20°, preferably by less than 10°, from the vertical direction.

The injection score or injection probability is indicative of a probability or likelihood that a particular identified dispense or ejection event originates from an actual injection event of the delivery device with the dispensed dose delivered to the patient. The injection score is the basis for ultimately assessing whether or not the second dispense event is acknowledged as a confirmed injection event. The injection score is aggregated from the responses to any number of criteria applied to a use cycle, by adding or otherwise suitably accumulating scores or weights for every fulfilled injection criteria or affirmative response. The injection criteria are mutually independent and may be individually weighted and aggregated or sequentially evaluated in a Decision Tree and/or based on manually defined priorities. The criteria applied may also be reflected in the architecture of a Neural Network, which is subsequently trained on a sufficiently large set of known use cycles with distinct sequences of dispense events, dispensed dose amounts, inter-event delays, and device orientations. Alternatively, or complementary, a priming or non-injection score may analogously be defined and exploited as detailed further below.

In a preferred embodiment, the method comprises increasing the injection score of the second dispense event if a multi-event-criteria is acknowledged, in particular if the second device orientation is different from the first device orientation, and/or if the second dispense dose amount is larger than the first dispense dose amount. These criteria reflect the assumption that the user is expected to prime in the upright position and to inject with a device orientation different from the upright position, and that an injection dose is generally larger than a preceding priming dose.

In a preferred embodiment, the method comprises increasing the injection score of the second dispense event if a cycle criteria is fulfilled, in particular if the second dispense event is the last dispense event in the cycle, and/or if a holding time following the second event is acknowledged. These criteria reflect the assumption that a user does not end the use cycle with a priming shot, but with an injection followed by observing an appropriate holding time before removing the injection device from the injection site.

In a preferred embodiment the method comprises, by the electronic unit and during the use cycle, determining further, in particular initial, event details including a dispense time, a dispense dose, and a device orientation of the device, of a further dispense event immediately preceding the first dispense event. Evaluating an injection criteria then comprises increasing the injection score of the second dispense event if a time interval between the further and the first dispense event time is smaller than a time interval between the first and the second dispense event time. This reflects the assumption that a time interval between successive priming events is smaller than the time interval between the last priming event and the first injection event, which may include further preparation activity or a mere hesitation by the user.

In a preferred embodiment the aforementioned criteria are complemented by single event criteria based on the second event details, including increasing the injection score if the second dispense event is not preceded by a knocking or tapping movement of the delivery device indicative of a user attempting to release air bubbles adhering to an inner surface of the reservoir prior to an intended priming shot.

In advantageous variants of the invention, the cycle injection dose is determined at the end of the use cycle, by adding the event dispense doses of the dispense events with an injection score exceeding an injection threshold. The user is informed about the determined cycle injection dose by the electronic unit, or by a mobile device of the user, and preferably given the opportunity to manually adjust the determined cycle injection dose, or to confirm the determined cycle injection dose to be logged for therapy monitoring purposes. An injection or priming status of each dispense event may also be derived from the event injection score and signaled to the user, by visual, acoustic, or tactile signaling means, in real-time to assure and support an inexperienced user.

In advantageous variants of the invention, the electronic unit comprises a delivery device orientation sensor in form of an accelerometer which measures proper acceleration, which is the acceleration experienced relative to freefall for orientation sensing relative to the direction of gravity. The electronic unit is adapted to determine a dispensed dose amount from a number of mechanical feedbacks such as dose-unit dispense clicks generated by the variable-dose injection device proportionate to the dispensed dose amount. The mechanical feedback is sensed by the accelerometer, in addition to other mechanical sensors of the electronic unit, including a gyroscope sensor and a piezo contact microphone. In addition, the accelerometer for device orientation sensing may at the same time be used to detect device cap removal or cannula insertion.

According to another aspect of the invention, a delivery system includes a variable-dose delivery device and an electronic unit configured to perform a method of monitoring a use cycle or delivery process including a first, a second, and a third dispense event performed by means of the variable-dose delivery device. The method comprises determining a first, a second, and a third dispense dose amount dispensed by the delivery device, during, respectively, the first, the second, and the third dispense event. The method further comprises determining a first, a second, and a third device orientation assumed by the delivery device during, respectively, the first, the second, and the third dispense event. The method includes evaluating relative, or inter-event, priming criteria or priming conditions involving the third dispense amount and/or the third device direction to acknowledge the second dispense event as a priming event. Evaluating relative priming criteria involving dispense event data of subsequent dispense events results in a confirmed identification of the second dispense event as a priming event with increased certainty, albeit at the cost of an additional delay, as the subsequent dispense data is not instantaneously available. The invention is particularly helpful for a second, or intermediate, dispense event in-between a first, presumed priming event and a third, presumed injection event. Reverting to relative priming criteria is useful in case the absolute, or intra-event, priming criteria are not unambiguous, for instance in case the device orientation of a candidate priming event is not upright. In case of a confirmed priming event, the second dispense dose amount is disregarded when calculating and reporting an injected or net dose of the use cycle.

In preferred refinements of the invention, evaluation of the priming criteria involves generating and quantifying a priming score or priming probability indicative of a probability or likelihood that a particular identified dispense event originates from, an actual and true priming event of the delivery device. The priming score is the basis for ultimately assessing whether or not the second dispense event is acknowledged as a confirmed priming event. The priming score is aggregated from the responses to any number of priming criteria applied to a dispense event, by adding or otherwise suitably accumulating scores or weights for every fulfilled priming criteria or affirmative response. The priming criteria are mutually independent and may be individually weighted and aggregated or sequentially evaluated in a Decision Tree and/or based on manually defined priorities. The criteria applied may also be reflected in the architecture of a Neural Network, which is subsequently trained on a sufficiently large set of known use cycles with distinct sequences of dispense events, dispensed dose amounts, inter-event delays, and device orientations.

In an advantageous variant, the relative priming criteria include the following question involving third, or subsequent, dispense event data:
- Is the second device orientation different from the third device orientation? This criteria reflects the fact that the user is expected to prime in the upright position and to inject with a device orientation different from the upright position.
- Is the second dispensed dose amount smaller than the third dispensed dose amount? This criteria reflects the assumption that a priming dose is generally smaller than a subsequent injection dose.

In an advantageous variant, the relative priming criteria include the following question involving first, or preceding, dispense event data:
- Is the second device orientation the same as the first device orientation?
- Is the second dispensed dose amount smaller than or equal to the first dispensed dose amount? This criteria reflects the assumption that a user starts off with a conservatively large first priming dose amount, and ensues with a smaller, second priming dose amount in case of doubts about a complete removal of all air from the reservoir in the first priming event.

In an advantageous embodiment, a relative or inter-event priming criteria involving dispense times of the first, second, and third dispense event is evaluated, and a priming score of the second dispense event is increased if a preceding time interval or delay between the first and second dispense event time is smaller than a subsequent time interval between the second and third dispense time. This criteria reflects the assumption that a time interval between successive priming events is smaller than the time interval between the last priming event and the first injection event, which may include further preparation activity or a mere hesitation by the user.

In a preferred embodiment, absolute or intra-event priming criteria are evaluated based on second dispense data and further use-cycle data readily available at the end of the second dispense event. The absolute criteria or conditions may be applied to the second dispense event and verified instantaneously at the end of the second dispense event at the latest. A or the priming score is aggregated from the responses to any number of absolute priming criteria, by adding or otherwise suitably accumulating scores or weights for every fulfilled priming criteria or affirmative response. The absolute priming criteria include the following questions:
- Is the second device orientation predominantly upright?
- Is the second dispense dose amount different from any dose of a therapy plan of the patient?
- Is the second dispensed dose amount below a predetermined threshold, wherein the predetermined threshold is a maximum recommended priming dose amount of preferably four dose units?
- Is the second dispense event preceded by a knocking or tapping movement of the delivery device indicative of a user attempting to release air bubbles adhering to an inner surface of the reservoir?
- Is the second dispense event preceded by a tilting movement of the delivery device towards an upright orientation?

The priming score may be assessed to provisionally mark or elect the second dispense event as a candidate priming event, to be confirmed or rejected in a post-event plausibility check or holistic use cycle review. An incorrect preliminary classification of the second dispense event due to, for instance, non-conformant user handling and corresponding second dispense event data, may thus be amended through the above evaluation of inter-event criteria.

In a preferred refinement the electronic unit includes a signaling element for acoustic, tactile, and/or optical signaling of a priming device status to the user. The priming device status is assessed from a priming score during the second dispense event, and signaled in real-time to assure and support an inexperienced user by confirming that the electronic unit has acknowledged the second dispense event as a priming event.

The method according to the invention is particularly useful to asses identified intermediate dispense events which may originate from either a last priming event or a first injection event performed by the delivery device. In the case of three dispense events, the priming score of the second dispense event is assessed to acknowledge the second dispense event as a priming event, or as an injection event, with the first and third dispense event being acknowledged as priming and injection events, respectively, and unless optional further evaluations lead, for instance, to a low priming score for the first dispense event. In case of four or more dispense events in a single use cycle, two or several triplets including three successive dispense events may be formed and subjected to the evaluation as outlined above. A last priming event preceding skin contact and followed by a first injection event may be acknowledged with reasonable certainty from one of the triplets, and further complemented by likewise acknowledging all earlier dispense events as priming events.

In advantageous variants of the delivery system, the electronic unit comprises a delivery device orientation sensor in form of an accelerometer which measures proper acceleration, which is the acceleration experienced relative to freefall for orientation sensing relative to the direction of gravity. Changes in the orientation or position of the delivery device may also be sensed by a gyroscope sensor, which in the first place serves as a mechanical sensor for detecting mechanical feedbacks such as dose-unit dispense clicks generated by the variable-dose injection device proportionate to the dispensed dose amount.

In a preferred variant, the electronic unit is part of an electronic module that in turn is adapted to be releasably attached, or detachably mounted, to a main housing or body of the injection device. The electronic module includes attachment means for securing or even locking the module to the device housing, such as biased protrusions engaging with counterpart recesses of the device housing. The electronic module is not mounted to a dose dialing or injection triggering part of the injection device, and not intended to be rotated or otherwise moved by the user relative to the main housing. The electronic module is adapted to determine a dispensed dose amount from a number of feedback events generated by the variable-dose injection device proportionate to the dispensed dose amount. The electronic module is attached to the device housing in a sufficiently rigid manner to enable mechanical feedback events such as dose-unit dispense clicks to be conveyed or transmitted to the electronic module and to feedback sensors of the electronic unit without excessive attenuation or distortion. The feedback sensors include the accelerometer, in addition to other mechanical sensors including a gyroscope and a piezo contact microphone, to determine dispense dose amounts.

Any electronic unit part of an electronic variable-dose delivery device or of an electronic module or auxiliary device for removable attachment to a mechanical variable-dose delivery device and adapted to determine a dispensed dose amount from a number of feedback events generated by the variable-dose injection device proportionate to the dispensed dose amount, may benefit from one or several of the following aspects. These aspects are independent from the specific post-event plausibility check or holistic use cycle review of the invention, for instance, determination of injected and priming doses of a use cycle may be performed in any possible manner.

The determined injected or net dose of the use cycle is ultimately transmitted to a mobile device or smartphone of the patient and/or to other stakeholders. In order to keep track of the amount of remaining medication in the reservoir, a total of all dispense dose amounts in the use cycle is stored by the electronic unit or likewise transmitted to the mobile device of the user. Alternatively, the dispense dose amounts of the priming events in the use cycle may be reported along with the net dose. The electronic unit or the mobile device aggregates the dispense dose amounts of all the use cycles since the last replacement of the reservoir, and signals a remaining amount of medication in the reservoir, preferably in a rather coarse manner limited to full, half, and close to empty, or otherwise alerts the user to prepare for reservoir replacement.

In case a patient follows a strict therapy with a predetermined delay between any two successive use cycles or delivery processes, or at least between two specific use cycles, the electronic unit may issue a double-injection warning if an actual inter-cycle delay is about to fall below the predetermined delay. A time stamp based on a local clock or counter of the electronic unit is saved for every use cycle completed. To avoid complications due to loss or unavailability of the local clock or counter, at least a latest absolute reference time received from the mobile device is likewise time stamped and stored. This supports a correct determination of the time elapsed between two successive use cycles, even if the local clock has been restarted in between.

The electronic unit implements a holding time function from a configurable timer. Whenever a dispense click or other feedback is recorded the timer is re-started. As soon as the timer reaches a predefined holding time, the user is notified that delivery device may now be safely removed from the injection site.

The user may dispense a dose too fast, with a frequency of the dispense clicks or feedbacks to be recorded exceeding a threshold, and leading to an impaired or even incorrect detection of the injected units. The user is warned after completion of the dose injection, that a) the last injection may not have been acknowledged properly and b) that the user reminded to inject slower next time.

In a particular delivery system, the injection device and the electronic module may be mounted in two orientations relative to each other, that is, the module or the device may be rotated by 180° around the longitudinal axis and still mechanically couple to each other. The injection device and the electronic module each have a single RFID antenna arranged essentially parallel to the longitudinal axis, wherein the two antennas present a comparable inductive coupling in both rotational mounting positions. A single RFID tag reader antenna thus inductively couples to an RFID tag antenna in either mechanical coupling position. The two antennas preferably each comprise an essentially flat antenna area arranged on lateral sides of the module and the device, and oriented perpendicularly to each other in the mounted state.

### BRIEF DESCRIPTION OF THE DRAWINGS

The subject-matter of the invention will be explained in more detail in the following text with reference to preferred exemplary embodiments as illustrated in the attached drawings, of which
Fig. 1 depicts a medical injection monitoring and patient support system;
Fig.2 summarizes the steps of the post-cycle dispense event identification; and
Fig.3 shows a cross section of an injection device with a concentric electronic module.

For consistency, the same reference numerals are used to denote similar elements illustrated throughout the drawings.

### DETAILED DESCRIPTION OF PREFERRED EMBODIMENTS

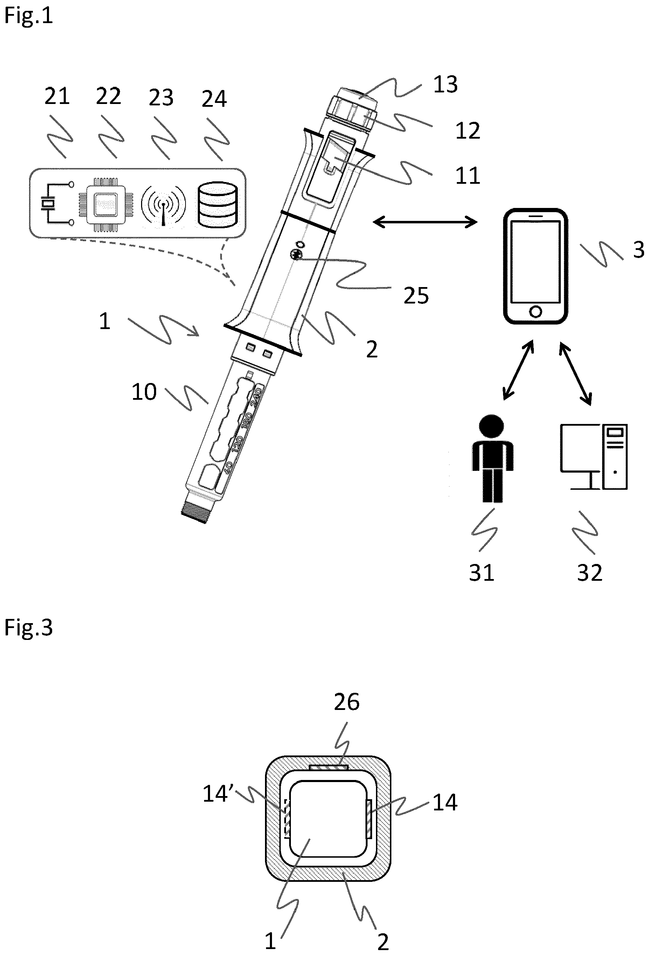

Fig.1 depicts a medical injection or drug delivery monitoring and patient support system, comprising a variable dose injection device 1, an electronic module 2 detachably mounted, or releasably attached, to the injection device, and a mobile device 3. The injection device includes an elongate device housing 10 essentially symmetric around a main device axis, as well as a dose dialing facility as amply described for instance in EP 2812055 and in turn including a dosing sleeve 11, a rotary dosing knob 12, and a discharge button 13. The dosing knob enables the user to adjust a dose and is arranged on a proximal end of the dosing sleeve. The dosing sleeve features markings in the form of numbers on its outer surface. When the dosing sleeve is screwed out of the housing during the dosing operation, the adjusted dose is displayed in a window of the housing. The discharge button is snapped on the dosing sleeve in such a way that the discharge button can slightly move axially relative to the dosing sleeve.

The electronic module 2 comprises an essentially tubular module housing surrounding the injection device housing in the attached state, a feedback sensor 21, a processing unit 22 and a transmitter unit 23 for wireless transmission of data about the injection progress via Bluetooth Low Energy (BLE) or equivalent short or near range wireless communication technology to the mobile device. The electronic module additionally includes a data storage unit 24 or memory connected to the processing unit and a lock/release mechanism to secure the attachment of the electronic module to the injection device. Connection and system status indicator 25 provides visual feedback about a connection status indicative of an established communication link to the mobile device, and about a device, module, or process status including for instance an availability of battery power, a readiness of communication means, an attached/detached status of the electronic module and the injection device, or a progress of an ongoing injection process. The electronic module is further adapted to produce a time-stamp indicating at what date and time a monitored dose has been injected, and to store at least the dose expelled and the time-stamp in the data storage unit for later upload.

The mobile device 3 is a smartphone or tablet device running a dedicated application program; or a laptop computer configured accordingly. The mobile device is adapted to interact with a respective patient 31 as well as a remote server, cloud based computing facility, or expert system 32. The mobile device may comprise a bolus calculator for calculating insulin delivery doses, or be knowledgeable of therapy plan of a growth hormone therapy. Instantaneous bolus dose values or currently scheduled therapy dose values may be provided to the electronic module. Dialed doses may then be monitored in real time and compared to the dose values due, and a discrepancy signaled to the user via indicator 25.

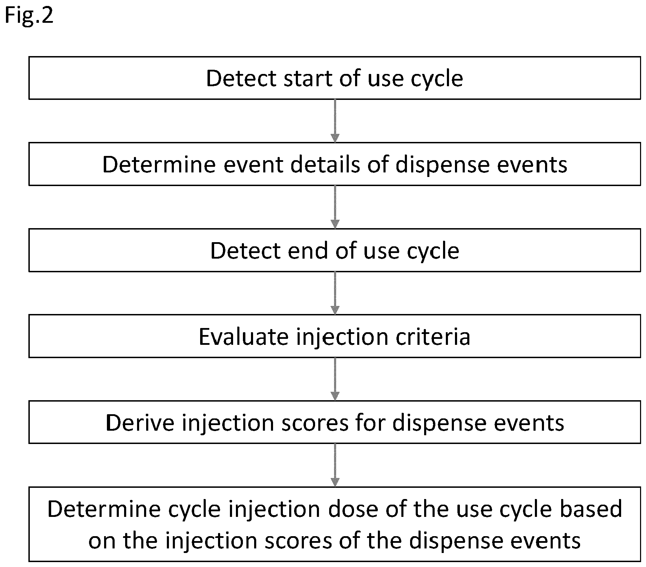

Fig.2 is a flow chart summarizing the essential steps of the method including post-cycle dispense event identification and cycle injection dose determination. Exemplary injection criteria to be evaluated in step 4 are listed in the following table, along with a rationale and/or conclusion for the present, or latest, dispense event being investigated.

| # | Criteria | Conclusion for the latest dispense event |
|---|---|---|
| 1 | Relative device orientation | A change in device orientation with respect to the preceding dispense event is indicative of a change from a preceding priming event to a present injection event |
| 2 | Relative dose size | As successive priming doses are hardly increasing, an observed increase in dispense event dose points to a change from a preceding priming event to a present injection event |
| 3 | Time between events | An increased inter-event time delay may be occasioned by the user preparing to inject, and is thus indicative of a change from preceding priming events to an injection event |
| 4 | Number of dose-units | Priming is recommended with 2 or 3 dose-units. A higher number is indicative of an injection event |
| 5 | Last dispense event of the use cycle | The last dispense event before re-capping the device is most probably an injection event |
| 6 | Priming cone | A dispense event with a device orientation within a priming cone is probably a priming event |
| 7 | New device | The first dispense event with a new device or reservoir is most probably a priming event |
| 8 | Dispensed dose according plan | A dispensed dose according the therapy plan (and other than 2 or 3 units) is indicative of an injection event |
| 9 | Holding time | An unchanged position of the device for a minimum holding time after the end of the product dispense is indicative of an injection event |
| 10 | Needle removal | Removal or extraction of the needle device from the injection site and/or removal of the needle from the device |
| | | (as detected in the response pattern of an accelerometer, gyroscope or other device movement sensor / Inertia Measurement Unit) after the dispense event is indicative of an injection event |
| 11 | Number of events | If there are more than two events in the use cycle, the last event is probably an injection event |

The criteria are assigned a weight or relative score that is involved when aggregating the results of the individual criteria evaluated into a final injection score. Each weight is indicative of a relevance or importance of a criteria, and may be determined by training on a large set of use cycles. Independent weights may be assigned for a criteria being fulfilled and for the criteria not being complied. The following table lists a selection of criteria with exemplary weights. The scores for the second dispense event of a sample use cycle with a priming event and a subsequent injection event of 4 or 5 dose units, both in upright direction, and followed by a holding time and needle removal, are reported in the last column. Despite the major weight attributed to the upright orientation of the device during the second event, the plurality of further criteria applied succeed in reversing the corresponding bias, such that the negative total score of the second event is correctly interpreted as an injection event.

| **Criteria** | Question: | Weight Yes | Weight No | Sample Score |
|---|---|---|---|---|
| **6** | Device held upright during the event? | 90 | -70 | 90 |
| **4** | 1-2 dose units in the event? | 50 | 0 | 0 |
| | 3 dose units in the event? | 30 | 0 | 0 |
| | 4-5 dose units in the event? | -20 | 0 | -20 |
| | 6-10 dose units in the event? | -50 | 0 | 0 |
| | 11-60 dose units in the event? | -95 | 0 | 0 |
| **9** | Holding time detected after event? | -20 | 40 | -20 |
| **10** | Cannula removal detected after the event? | -20 | 10 | -20 |
| **5** | Last event in the use cycle? | -70 | 60 | -70 |
| **1** | Device orientation w.r.t. previous event? | -30 | 20 | 20 |
| **3** | Inter-event time delay has increased? | -20 | 0 | 0 |
| **11** | More than 2 events in the use cycle? | -20 | 0 | 0 |
| | Sum | | | **-20** |

Fig.3 discloses a cross-sectional view of an injection device 1 with an RFID tag or transponder and an electronic module 2 with an RFID tag reader surrounding the injection device 1. The RFID tag comprises a tag antenna 14 and the RFID tag reader comprises a reader antenna 26, both with an antenna area essentially parallel to the longitudinal axis of the injection device. The antenna areas are preferably made of a conductive track on a flexible circuit board which forms a coil defining the active antenna area with an extension or diameter between 2 and 3 cm. The antennas 14, 26 are generally arranged as close as possible to each other to allow for optimal inductive coupling. The RFID reader of the reusable electronic module may read an ID of the disposable injection device, alphanumeric drug information, and/or further information including an encryption key for securing communication between the electronic module and a user device. In the embodiment depicted the two antennas 14, 26 are planar or two-dimensional coils rotated by 90° with respect to each other, such that the antennas are perpendicular to each other and such that there is no immediate overlap of the two antenna areas. The four-fold rotational symmetry due to the square-shaped cross section is further reduced by guiding elements on the surface of the injection device that only allow two different orientations of the electronic module with respect to the injection device, i.e. the injection device may be rotated by 180° around the longitudinal axis and still fit into the electronic module. In this rotated position, the tag antenna is placed in position 14' as indicated in broken lines, and has a similar inductive coupling to the reader antenna 26 as in the first orientation.

Turning the dosing knob of the injection device in a dose-increasing dialing direction or in a dose-reducing corrective direction causes a ratchet toothing to slide over a counterface toothing and to repeatedly perform a slight axial back and forth motion that gives rise to a clicking sound. The number of clicks is proportional to the dosage volume, wherein preferably each click or vibration burst corresponds to a single dosage unit, such as an International Unit IU. During dose-discharge, a tooth of a flexible arm rotates relative to a grating, which in turn generates an acoustic click sound and a tactile discharge feedback signal to the user. The dialing, corrective, and discharge clicks are captured by the feedback sensors and converted into a feedback signal on behalf of the processing unit.

The module housing is designed to be positioned on the injection device housing in such a way as to neither interfere with the dial-and-dose components nor obscure any display window or visual indicator of the device. To this purpose, the module housing has a recess or opening that matches with the window. Hence the patient may continue using a non-modified injection device in a known manner, despite the presence of the electronic module, with all device interface elements remaining fully accessible throughout the handling sequence. Specifically, in this case the electronic module excludes the presence of a mechanical sensor to mechanically detect a rotation angle or linear shift of the dosing knob. Likewise, the electronic module excludes the presence of an optical sensor to read a dialed dose from a dialing sleeve.

The electronic module comprises an accelerometer and/or a gyroscope as a feedback sensor for dose detection. The signal processing unit is adapted to detect device handling activities by the user from a non-click sensor signal measured independent of a dose setting or dose expel process of the delivery device, and not related to a feedback event generated by feedback means internal to the delivery device. Device handling activities relate to movements of the delivery device and/or the electronic module as a whole, and include, for instance, attachment/removal of the module to/from the delivery device by way of a snap or clip operation; seizure of delivery device and attached module after having rested immobile for some time, device cap attachment/removal to/from the delivery device, cannula attachment or removal. Detection of the aforementioned user activities may advantageously be used, individually or in combination, as a basis for a plausibility check confirming or questioning an expected handling sequence. Upon detection of these activities, the electronic module may end a sleep or low-power mode and enter an injection monitoring mode, and/or identify the start of a use cycle. Likewise, a dedicated wake-up activity such as double tapping or knocking the device to end a sleep mode may be detected by the same feedback sensor that primarily serves for dose determination. In these cases, no button or switch may be needed on the electronic module to activate the electronic module. Alternatively, a touch sensor may be used to wake up the electronic unit and identify the start of the use cycle.

The electronic components being integrated in the injection device or being part of an electronic module may comprise a visual, audible and/or tactile status indicator indicating to a user a status of the system. The status indicator may explicitly exclude any advanced human-machine interfacing capability, and be limited to a few, specifically less than ten, messages conveyable to the user. In particular, the electronic unit may not be wired to, and the electronic module may be devoid of, a display, screen, or projector for visually transmitting readable instructions, and likewise exclude an artificial speech assistant for reading out loud the instructions. Such advanced HMI functionality including elaborate graphic display and speech output capabilities are preferably being provided by a mobile device communicatively connected to the electronic unit. The status information may be redundant or complementary to primary signals from the injection device that a user may still capture in parallel. In particular, the status information may include a positive confirmation of a dose having been set or corrected, or an indication about a lapse of a minimum holding, delay, or dwell time following completion of a substance expel or piston forwarding activity to inform the user that it is now safe to remove the injection device.

While the invention has been described in detail in the drawings and foregoing description, such description is to be considered illustrative or exemplary and not restrictive. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims shall not preclude the existence of further meaningful combinations of these elements or steps.

### LIST OF REFERENCE NUMERALS

- 1: injection device
- 10: device housing
- 11: dosing sleeve
- 12: dosing knob
- 13: discharge button
- 14: tag antenna
- 2: electronic module
- 20: module housing
- 21: feedback sensor
- 22: processing unit
- 23: transmitter unit
- 24: data storage unit
- 25: status indicator
- 26: reader antenna
- 3: mobile device
- 31: patient
- 32: data server

## Claims

1. A method of monitoring a use cycle including a first and a second dispense event performed by means of a variable-dose delivery device, comprising, by an electronic unit associated with the delivery device,
- determining, during the use cycle, first and second event details of the first and second dispense event, respectively,
- determining, at the end of the use-cycle, cycle properties of the use-cycle, and
- evaluating, after the use cycle, an injection criteria to derive an injection score of the second dispense event, indicative of a probability that the second dispense dose has been injected into the target tissue, wherein the injection criteria is a multi-event criteria based on an event detail of the first event or a cycle criteria based on a cycle property of the use cycle, and
- determining a cycle injection dose based on the injection score.

2. The method of claim 1, wherein the first and second event details include a first and a second device orientation as well as a first and a second dispense dose amount, respectively, and wherein evaluating the multi-event criteria comprises increasing the injection score of the second dispense event if
- the second device orientation is different from the first device orientation, and/or
- the second dispense dose amount is larger than the first dispense dose amount.

3. The method of claim 1 or 2, wherein evaluating the cycle criteria comprises increasing the injection score of the second dispense event if
- the second dispense event is the last dispense event in the cycle, and/or
- a holding time following the second event is acknowledged.

4. The method of claim 2 or 3, wherein the first and second event details include a first and a second dispense event time, comprising determining further event details including a further dispense event time of a further dispense event preceding the first dispense event, and wherein evaluating the multi-event criteria comprises increasing the injection score of the second dispense event if
- a time interval between the further and the first dispense event time is smaller than a time interval between the first and the second dispense event time.

5. The method of claim 2 to 4, wherein evaluating the injection criteria comprises increasing the injection score of the second dispense event if
- the second device orientation of the delivery device is not upright,
- the second dispense dose amount does match a dose according to a therapy plan,
- the second dispensed dose amount is above a threshold, and/or
- the second dispense event is not preceded by a knocking or tapping movement of the delivery device.

6. The method of one of claims 1 to 5, comprising, at the end of the use-cycle and by the electronic unit or by a mobile device of the user,
- signaling the determined cycle injection dose to a user, and
- logging the user-confirmed or adjusted cycle injection dose.

7. The method of one of claims 1 to 5, wherein the electronic unit comprises an accelerometer as a delivery device orientation sensor for determining the device orientation, comprising
- sensing, by the accelerometer, mechanical feedbacks of the delivery device, and determining, by the electronic unit, a dispense dose amount based on the mechanical feedbacks.

8. A delivery system including a variable-dose delivery device and an electronic unit for monitoring a use-cycle including a first, a second, and a third dispense event performed by means of the delivery device, the electronic unit being configured to
- determine a first, a second, and a third dispense dose amount dispensed, as well as a first, a second, and a third device orientation assumed by the delivery device, during the first, the second, and the third dispense event, respectively, and
- evaluate a relative priming criteria involving the third dispense amount and/or the third device direction to acknowledge the second dispense event as a priming event.

9. The delivery system of claim 8, wherein the electronic unit is configured to evaluate the relative priming criteria by increasing a priming score of the second dispense event if
- the second device orientation is different from the third device orientation, and/or
- the second dispensed dose amount is smaller than the third dispensed dose amount.

10. The delivery system of claim 8, wherein the electronic unit is configured to evaluate the relative priming criteria by increasing a priming score of the second dispense event if
- the second device orientation is the same as the first device orientation, and/or
- the second dispensed dose amount is not larger than the first dispensed dose amount.

11. The delivery system of claim 8, wherein the electronic unit is configured to determine a first, a second, and a third dispense event time, and to evaluate the relative priming criteria by increasing a priming score of the second dispense event if a time interval between the first and second dispense event time is smaller than a time interval between the second and third dispense time.

12. The delivery system of claim 8, wherein the electronic unit is configured to evaluate absolute priming criteria involving the second dispense amount and/or the second device direction, and/or further use-cycle information available at the end of the second dispense event, and to evaluate the relative priming criteria by increasing a priming score of the second dispense event if
- the second device orientation of the delivery device is upright,
- the second dispense dose amount does not match a dose according to a therapy plan,
- the second dispensed dose amount is below a threshold, and/or
- the second dispense event is preceded by a knocking or tapping movement of the delivery device.

13. The delivery system of any of claims 9 to 12, wherein the electronic unit is configured to
- assess the priming score of the second dispense event, and to
- signal a priming status of the delivery device to the user.

14. The delivery system of any of claims 9 to 12, wherein the electronic unit comprises an accelerometer as a delivery device orientation sensor for determining the device orientation.

15. The delivery system of claim 14, wherein the electronic unit is part of an electronic module comprising attachment means for releasable attachment of the electronic module to a main housing part of the variable-dose delivery device, and wherein the electronic unit comprises a feedback sensor to determine a dispense dose amount from mechanical feedbacks of the delivery device, and wherein the feedback sensor includes the accelerometer.
